# EUROPEAN PATENT APPLICATION

(11) **EP 4 360 701 A1**
(43) Date of publication of application: **01.05.2024**
(21) Application number: 21947018.4
(22) Date of filing: 22.06.2021
(51) Int. Cl.: A61N 5/06, A61N 5/067

(54) **LIGHT IRRADIATION DEVICE AND CONTROL METHOD FOR LIGHT IRRADIATION DEVICE**

(71) Applicant: Shimadzu Corporation, Nakagyo-ku, Kyoto-shi, Kyoto 604-8511 (JP)
(72) Inventor: ISHIKAWA, Akihiro, Kyoto-shi, Kyoto 604-8511 (JP)
(74) Representative: Müller-Boré & Partner Patentanwälte PartG mbB
(86) International application number: PCT/JP2021/023495
(87) International publication number: WO 2022/269730

(57) **Abstract**

A controller (30) of this light irradiation device (100) is configured to control a plurality of light guide members (20) connected to a plurality of light output ports, respectively, in an identifiable manner by causing the identification light (90) to be emitted from each of the plurality of light output ports (12).

## Description

### Technical Field

The present invention relates to a light irradiation device and a control method for a light irradiation device.

### Background Art

Conventionally, a light irradiation device is known for use in a therapeutic technique in which therapeutic light is emitted to a treatment target site of a subject to which a medicine containing a photosensitive substance has been administered. Such a device is disclosed in, for example, International Publication No. WO 2021-038913.

International Publication No. WO 2021/038913 described above discloses a light irradiation device for use in photoimmunotherapy. Photoimmunotherapy is a therapeutic method in which a medicine containing a fluorescent material that causes a photochemical reaction and an antibody that selectively binds to a cancer cell is administered into a body of a cancer patient, and the cancer cells are irradiated with light in a specific wavelength range corresponding to the fluorescent material to generate a photochemical reaction to thereby destroy the cancer cells. The light irradiation device is equipped with an excitation light source for emitting light in a specific wavelength range corresponding to a fluorescent material (hereinafter referred to as "therapeutic light") and an optical fiber for guiding the therapeutic light to the cancer cells.

### Prior Art Document

### Patent Document

Patent Document 1: International Publication No. WO 2021/038913

### SUMMARY OF THE INVENTION

### Problems to be Solved by the Invention

Although not disclosed in International Publication No. WO 2021/038913, there are cases where a plurality of optical fibers is used to guide therapeutic light from an excitation light source, depending on the size of the treatment target site. The optical fiber is a long, thin, cable-like light guiding member drawn from the light output port of the excitation light source to the patient. Therefore, when using a plurality of light guide members, it may sometimes become difficult to identify which light guide member is connected to which light output port because the plurality of light guide members becomes entangled with each other between the light output ports and the patient. In cases where it is not known which light guide member is connected to which light output port, it becomes difficult to set the intensity of the therapeutic light emitted from each light guide member and adjust the intensity of the therapeutic light to match the set value.

Further, the therapeutic light emitted from the light guiding member is high-intensity (high power) laser light, and the operator involved in the treatment need to wear light-shielding glasses for shielding the light in the wavelength range including the therapeutic light to protect his/her eyes from the laser light. Therefore, it is not easy to visually recognize the therapeutic light through the light-shielding glasses even if the therapeutic light is emitted from the light guide member to check the connection with the individual light output ports. Therefore, complicated work to untangle the plurality of light guide members is required before the treatment, which is an inconvenience that lengthens the preparation time required until the start of the treatment.

The present invention has been made to solve the above-described problems. One object of the present invention is to provide a light irradiation device and a control method for the light irradiation device capable of easily identifying which light guiding member is connected to which light output port.

### Means for Solving the Problems

In order to achieve the above-describe object, a light irradiation device according to a first aspect of the present invention is a light irradiation device for irradiating a treatment target site of a subject to which a medicine containing a photosensitive substance has been administered with therapeutic light, the light irradiation device comprising:
a light source including a plurality of light output ports, the light source being configured to emit the therapeutic light and identification light from each of the plurality of light output ports, the identification light being lower in intensity than the therapeutic light;
a plurality of light guide members each having an incident end to be connected to one of the plurality of light output ports of the light source and an emission end to emit light incident from the incident end; and
a controller configured to control the light source,
wherein the controller is configured to perform control to cause the identification light to be emitted from each of the plurality of light output ports to light up the plurality of light guide members connected to the plurality of light output ports, respectively, in an identifiable manner.

A control method for a light irradiation device according to a second aspect of the present invention is a control method for a light irradiation device for irradiating a treatment target site of a subject to which a medicine containing a photosensitive substance has been administered with therapeutic light, the control method comprising:
a step of causing identification light lower in intensity than the therapeutic light to be emitted from each of the plurality of light output ports to which the plurality of light guide members is connected, respectively, to light up the plurality of light guide members connected to the plurality of light output ports in an identifiable manner; and
a step of causing the therapeutic light to be emitted from each of the plurality of light output ports.

### Effects of the Invention

According to the present invention, as described above, the control to light up a plurality of light guiding members connected to the plurality of light output ports, respectively, in an identifiable manner by causing identification light lower in intensity (lower in power) than the therapeutic light to be emitted from each of the plurality of light output ports. For example, the identification light is emitted separately from the plurality of light output ports one by one, or the identification light different in lighting pattern (e.g., blinking speed) is emitted from the plurality of light output ports simultaneously. With this, it is possible to confirm that the identification light from each of the light output ports is emitted from which of the plurality of light guide members. At this time, from the light guide member, the identification light lower in intensity than the therapeutic light is emitted, so that the identification light can be confirmed without the use of light-shielding glasses. As a result, even in a case where a plurality of light guide members remains entangled, it is possible to easily identify which light guide member is connected to which light output port by the identification light. Further, with this configuration, it becomes possible to shorten the preparation time required before initiating the treatment.

### BRIEF DESCRIPTION OF THE DRAWINGS

FIG. 1 is a schematic configuration diagram of a light irradiation device according to one embodiment.
FIG. 2 is a schematic diagram showing a state in which identification light is caused to be emitted from a light guide member connected to a light source.
FIG. 3 shows a schematic diagram showing a forward emission type light guide member (A) and a circumferential emission type light guide member (B).
FIG. 4 is a block diagram showing a configuration of a light source unit equipped by a light source.
FIG. 5 is a schematic perspective diagram showing light-shielding glasses.
FIG. 6 is a block diagram for explaining a configuration of a controller of a light irradiation device.
FIG. 7 is a schematic diagram showing one example of an operation reception screen displayed on a display unit.
FIG. 8 is a timing chart showing one example of a lighting-on/lighting-off pattern of identification light at each light output port.
FIG. 9 is a flowchart showing a control method for a light irradiation device.
FIG. 10 is a flowchart showing output processing of identification light.
FIG. 11 is a schematic diagram of a modification in which a first light source generates therapeutic light (A) and therapeutic light (B).

### EMBODIMENTS FOR CARRYING OUT THE INVENTION

Hereinafter, some embodiments in which the present invention is embodied will be described based on the attached drawings.

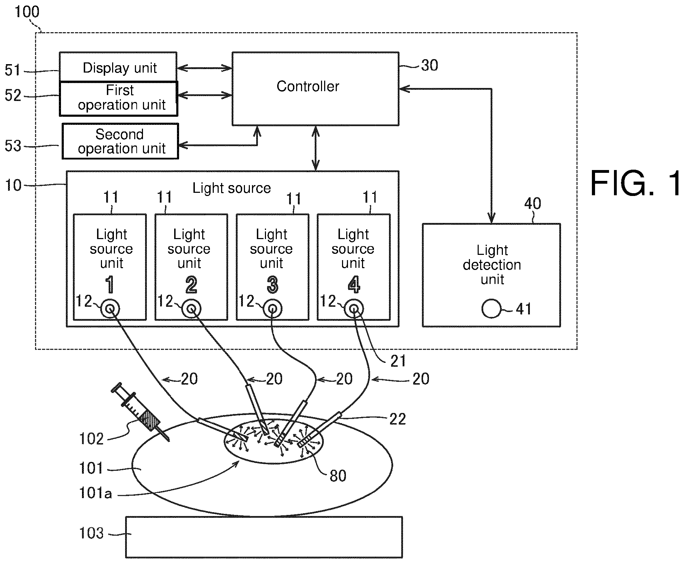

Referring to FIG. 1 to FIG. 8, a configuration of a light irradiation device 100 according to one embodiment of the present invention will be described.

The light irradiation device 100 shown in FIG. 1 is a light irradiation device for irradiating the treatment target site 101a of a subject to which a medicine containing a photosensitive substance has been administered with therapeutic light 80. In this embodiment, the light irradiation device 100 used for photoimmunotherapy will be described.

Photoimmunotherapy is a therapeutic method in which a medicine containing a fluorescent material that causes a photochemical reaction and an antibody that selectively binds to a cancer cell are administered into the body of a cancer patient, and the cancer cells are irradiated with light in a specific wavelength range corresponding to the fluorescent material to generate a photochemical reaction to thereby destroy the cancer cells.

In therapeutic photoimmunotherapy, a medicine 102 is administered into the body of a cancer patient 101 before the treatment. The medicine 102 contains a complex of a fluorescent material that emits fluorescence and an antibody. The cancer patient 101 is one example of the "subject" recited in claims. Further, the cancer patient 101 may be an animal other than a human being.

The fluorescent material is a substance that causes a photochemical reaction by being continuously irradiated with light in a specific wavelength range. In a photoimmunotherapy treatment (a treatment to kill cancer cells based on irradiating a medicine 102 containing a fluorescent material with light in a specific wavelength range), the therapeutic light 80 in a specific wavelength range in which the fluorescent material corresponding to the type of the fluorescent material in the medicine 102 administered to the cancer patient 101 causes a photochemical reaction is emitted to the treatment target site 101a of the cancer patient 101 by the light irradiation device 100. The treatment target site 101a is, for example, but not explicitly limited to, a head and neck, a chest, an abdomen, a back, or an organ in the body (e.g., a digestive tract, a liver, an adrenal gland, etc.).

Further, the fluorescent material is a substance that emits fluorescence when excited by being irradiated with the therapeutic light 80. The fluorescent material is, for example, a chemical, such as, e.g., IRDye (registered trademark) 700DX (hereafter referred to as "IR700"). IR700 is excited by the light with a wavelength of 600 nm or more and 700 nm or less and emits light with a wavelength of about 700 nm or about 770 nm as fluorescence.

The therapeutic light 80 is light in the wavelength range in which the fluorescent material of the medicine 102 used for treatment causes a photochemical reaction and differs depending on the type of fluorescent material of the medicine used for treatment. In the case where IR700 is used as the fluorescent material, the therapeutic light 80 is light with a wavelength of 600 nm or more and 700 nm or less, for example, nonthermal red light (near-infrared light) with a wavelength of about 690 nm.

The antibody contained in the medicine 102 selectively binds to the antigen (a specific protein expressed on cancer cells) of cancer cells. Therefore, the antibody is selected according to the antigen of the cancer cells to be treated. The antibody is, for example, cetuximab.

The medicine 102 is administered in advance to the cancer patient 101 before emitting the therapeutic light 80. The medicine 102 administered to the cancer patient 101 circulates through the body of the cancer patient 101 by the blood flow and selectively binds to the antigen of the cancer cells present at the treatment target site 101a by the antibody. The fluorescent material in the medicine 102 absorbs light in a specific wavelength range according to the fluorescent material to cause a photochemical reaction, which changes the chemical structure of the fluorescent material. This change in the chemical structure of the fluorescent material causes a change in the steric structure of the antibody. And, the change in the steric structure of the antibody bound to the cancer cells causes damage to the cell membrane of the cancer cells. As a result, the cancer cells are swollen and ruptured by the water that has penetrated through the damaged portions of the membranes of the cancer cells, thereby destroying (killing) the cancer cells. Note that the fluorescent material will no longer emit fluorescence after causing a change in its chemical structure due to a photochemical reaction.

In this embodiment, the following description will be directed to near-infrared photoimmunotherapy (Near-Infrared Photoimmunotherapy: NIR-PIT), which uses, among other things, a medicine 102 using IR700, which is sensitive to near-infrared light, as the fluorescent material and near-infrared rays as the therapeutic light 80, but the near-infrared photoimmunotherapy is not limited thereto.

### (Configuration of Light Irradiation Device)

As shown in FIG. 1, the light irradiation device 100 is configured to output therapeutic light 80 in a specific wavelength range according to the fluorescent material contained in the medicine 102 used for photoimmunotherapy. The light irradiation device 100 is equipped with a light source 10, a plurality of light guide members 20, and a controller 30. Further, in the example shown in FIG. 1, the light irradiation device 100 is further equipped with a light detection unit 40, a display unit 51, a first operation unit 52, and a second operation unit 53.

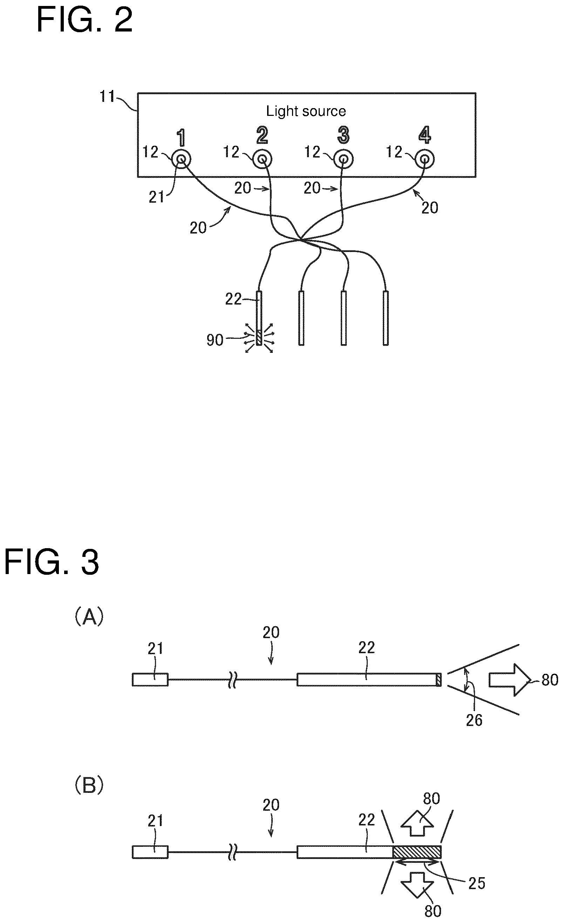

The light source 10 includes a plurality of light output ports 12. The light source 10 is configured to emit therapeutic light 80 and identification light 90 (see FIG. 2) from each of the plurality of light output ports 12.

The therapeutic light 80 is light of a first wavelength included in an absorption wavelength bandwidth of the fluorescent material contained in the medicine 102 administered into the body of the cancer patient 101 in the treatment in photoimmunotherapy. The therapeutic light 80 of the first wavelength is absorbed by the fluorescent material to excite the fluorescent material, thereby generating a photochemical reaction.

On the other hand, the identification light 90 (see FIG. 2) is used to identify the light guide members 20 connected to the plurality of light output ports 12, respectively. As will be described below, the light guide member 20 is a cable-like member that is drawn from the light output port 12 to the cancer patient 101 on the bed 103. Therefore, as shown in FIG. 2, the plurality of light guide members 20 connected to the plurality of light output ports 12, respectively, may sometimes be entangled with each other. In this case, it is difficult for the operator to determine which light guide member 20 is connected to which light output port 12. With the lighting-on of the identification light 90, the connection relation between the light guide member 20 and the light output port 12 is confirmed. The identification light 90 is light lower in intensity than the therapeutic light 80. In this embodiment, the identification light 90 is light of a second wavelength different from the first wavelength. In other words, the identification light 90 is light different in color from the therapeutic light 80.

The light output port 12 is an emission port for the therapeutic light 80 and the identification light 90, as well as a connection port for the light guide member 20. One light output port 12 is connected by one light guide member 20. The number of light output ports 12 is not expressly limited as long as it is plural. In the example shown in FIG. 1, an example is shown in which the light source 10 includes four light output ports 12. The plurality of light output ports 12 is each configured to emit light (the therapeutic light 80 and/or the identification light 90) to the incident end 21 of the light guide member 20 connected to the light output port 12. The plurality of light output ports 12 is each assigned by a port number. In the example shown in FIG. 1, the four light output ports 12 are distinguished from each other as "PORT 1" to "PORT 4."

The light source 10 is provided with a plurality of light source units 11. The plurality of light source units 11 is provided in the same number as the number of the light output ports 12, and each light source unit 11 has one light output port 12.

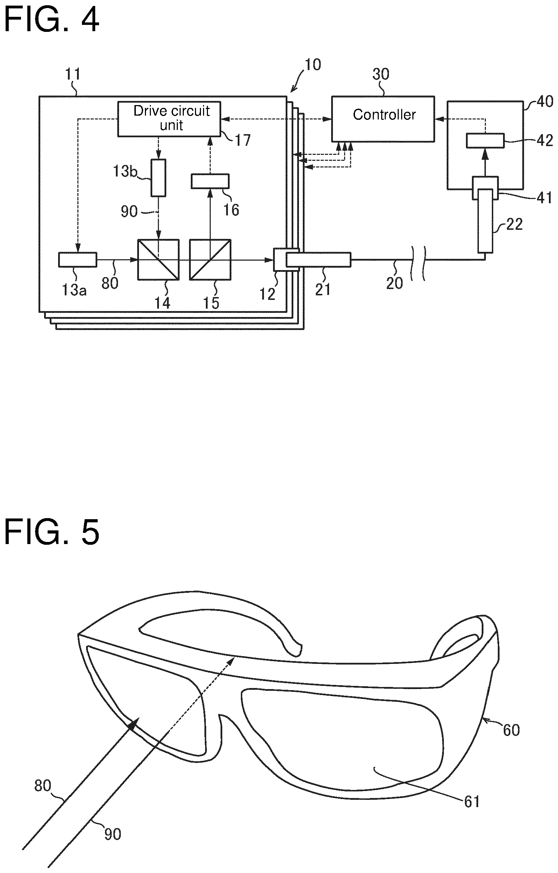

The plurality of light source units 11 each includes a first light source 13a (see FIG. 4) for generating therapeutic light 80 and a second light source 13b (see FIG. 4) for generating identification light 90 (see FIG. 2). The plurality of light source units 11 can emit the therapeutic light 80 and the identification light 90, independently. Therefore, the light source 10 is configured to emit the therapeutic light 80 and the identification light 90 independently from the plurality of light output ports 12. Further, the light source 10 can individually switch the emission and emission stop of the therapeutic light 80 from each of the plurality of light output ports 12. Similarly, the light source 10 is configured to individually switch the emission and emission stop of the identification light 90 from each of the plurality of light output ports 12.

The light guide member 20 is connected to any one of the light output ports 12 of the light source 10 and thus functions to guide the light emitted from the light output port 12 to the treatment target site 101a. The plurality of light guide members 20 is connected to the plurality of light output ports 12, respectively. The light guide member 20 is a disposable consumable. The maximum number of the light guide members 20 that can be simultaneously connected to the light source 10 is the number of the light output ports 12. The number of the light output ports 12 (i.e., the number of the light guide members 20) used simultaneously is determined according to the treatment content. In FIG. 1, a total of four light guide members 20 are connected to the four light output ports 12 one by one.

Each of the individual light guide members 20 has an incident end 21 to be connected to one of the plurality of light output ports 12 of the light source 10 and an emission end 22 for emitting light incident from the incident end 21. The light guide member 20 is an optical fiber cable connecting the incident end 21 and the emission end 22. The emission end 22 of the light guide member 20 is configured by a light diffusing member (light diffuser). There is a plurality of types of light guide members 20 depending on the type (structure) of the light diffusing member constituting the emission end 22. As shown in FIG. 3(A), one type of the light guide member 20 is a forward emission type that emits light in a cone-beam (or polygonal pyramid) shape by diffusing light at a predetermined irradiation angle 26 toward a forward direction perpendicular to the end face of the emission end 22. The forward emission type light guide member 20 is mainly used in applications in which the therapeutic light 80 is emitted from the outside of the body of the cancer patient 101 toward the body surface.

As shown in FIG. 3(B), the other type of the light guide member 20 is a circumferential emission type in which light is emitted in a ring (or cylindrical) shape by diffusing light from the outer circumference of a cylindrical light diffusion member toward a radial direction perpendicular to the outer circumference. The circumferential emission type light guide member 20 is mainly used in applications in which the emission end 22 is inserted into the body of the cancer patient 101 to irradiate the treatment target site 101a with the therapeutic light 80 from the inside of the body. FIG. 1 shows an example of a circumferential emission type light guide member 20 with the emission end 22 inserted into the body of the cancer patient 101.

As shown in FIG. 1, the controller 30 is a computer configured to control the light source 10. The controller 30 is configured to perform therapeutic light irradiation processing to control the light source 10 to cause the therapeutic light 80 to be emitted from the emission end 22 of the light guide member 20 at the time of treatment in photoimmunotherapy. The controller 30 can individually control the start of irradiation of the therapeutic light 80 and the stop of irradiation of the therapeutic light 80 (switching the irradiation ON/OFF of the therapeutic light 80) for each of the plurality of light output ports 12 by controlling the plurality of light source units 11, individually.

Further, in this embodiment, the controller 30 is configured to perform control (light guide member identification processing) to light up the plurality of light guide members 20 connected to the plurality of light output ports 12, respectively, in an identifiable manner by emitting the identification light 90 (see FIG. 2) from each of the plurality of light output ports 12. The controller 30 can individually control the start of emitting the identification light 90 and the stop of emitting the identification light 90 (switching emission ON/OFF of the identification light 90) for each of the plurality of light output ports 12 by individually controlling the plurality of light source units 11.

Further, the controller 30 is configured to perform calibration processing for adjusting the emission intensity of the therapeutic light 80 at each of the plurality of light output ports 12, based on the detection result of the light detection unit 40.

The light detection unit 40 is configured to accept the emission end 22 of any one of the plurality of light guide members 20 to detect the light (therapeutic light 80) output from the emission end 22. The light detection unit 40 outputs a signal corresponding to the detected light intensity to the controller 30. The light detection unit 40 includes a light input port 41 to which the emission end 22 is connected (inserted).

Further, the controller 30 is configured to control the screen display of the display unit 51 and perform control according to an operation input to the first operation unit 52 and the second operation unit 53.

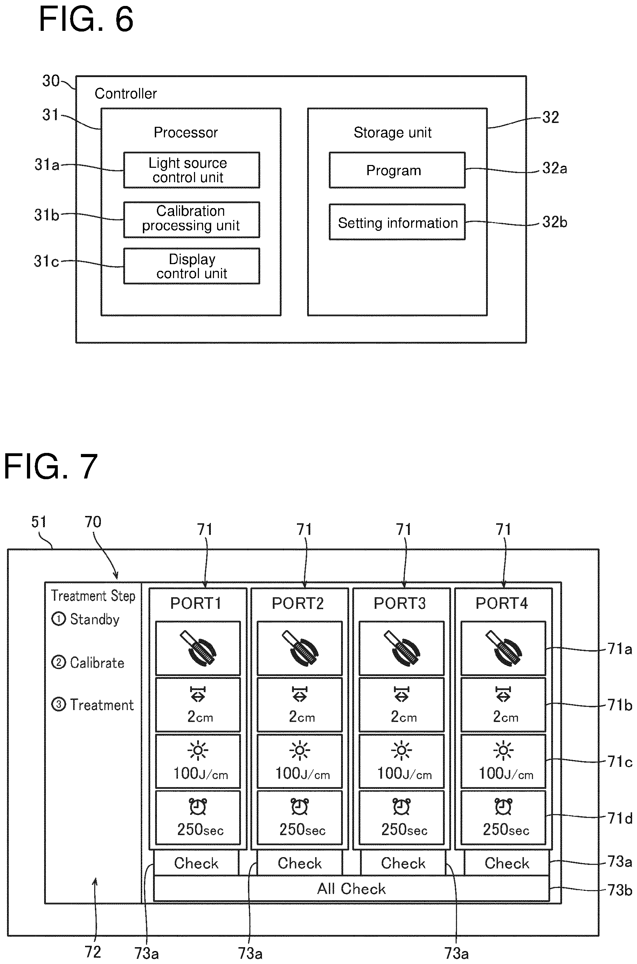

The display unit 51 displays a screen for inputting setting information 32b (see FIG. 6) of the light irradiation device 100, a screen for operating the light irradiation device 100, and other screens under the control of the controller 30. The display unit 51 is configured by a display device, such as a liquid crystal display and an organic EL display.

The first operation unit 52 receives an operation input to an input screen (GUI) displayed on the display unit 51. The first operation unit 52 is, for example, a touch panel provided on the display unit 51. In other words, the display unit 51 and the first operation unit 52 constitute a touch panel display. The first operation unit 52 outputs a signal corresponding to the received operation input to the controller 30.

The second operation unit 53 is connected to the controller 30 and is configured to receive an operation input of irradiation start and irradiation stop of the therapeutic light 80. The second operation unit 53 is configured by an input device, such as a foot switch and a hand switch.

### (Configuration of Light Source Unit and Light Detection Unit)

FIG. 4 shows one configuration of one of the plurality of light source units 11 provided by the light source 10. Since the configurations of the plurality of light source units 11 are identical, one light source unit 11 will be described here.

The light source unit 11 has a light output port 12, a first light source 13a, a second light source 13b, an optical element 14, an optical element 15, a light-receiving unit 16, and a drive circuit unit 17.

The first light source 13a and the second light source 13b are each configured by a laser light source. The first light source 13a and the second light source 13b are each configured, for example, by a semiconductor laser device. The first light source 13a outputs the therapeutic light 80 of a first wavelength. The second light source 13b outputs the identification light 90 of a second wavelength different from the first wavelength. The first light source 13a and the second light source 13b output the therapeutic light 80 and the identification light 90, respectively, toward the optical element 14.

The optical element 14 is configured to guide the light incident from the first light source 13a and the light incident from the second light source 13b to the optical element 15. In the example in FIG. 4, the optical element 14 is configured to transmit the therapeutic light 80 from the first light source 13a to be emitted to the optical element 15 and reflect the identification light 90 from the second light source 13b to be emitted to the optical element 15. The optical element 14 is configured by a mirror element, such as a dichroic mirror and a half mirror.

The optical element 15 is configured to split the light incident from the optical element 14 to be output to the light output port 12 and the light-receiving unit 16, respectively. In the example in FIG. 4, the optical element 15 is a beam splitter that transmits a part of the light incident from the optical element 14 to be emitted to the light output port 12 and reflects the other part of the light incident from the optical element 14 to be emitted to the light-receiving unit 16. The optical element 15 is configured by a mirror member, such as, e.g., a half-mirror.

The light-receiving unit 16 is configured to receive the light incident from the optical element 15 and output a signal corresponding to the received light intensity to the drive circuit unit 17. The light-receiving unit 16 is configured by a light sensor, such as, e.g., a photodiode. The light output port 12 is configured to output the light incident from the optical element 15 to the incident end 21 of the light guide member 20.

The drive circuit unit 17 controls the ON/OFF of the first light source 13a and the second light source 13b according to the control signal from the controller 30. The drive circuit unit 17 controls the light intensity output from each of the first light source 13a and the second light source 13b according to the control signal from the controller 30. The drive circuit unit 17 can control the first light source 13a and the second light source 13b independently and can also turn on the first light source 13a and the second light source 13b simultaneously. The drive circuit unit 17 drives the first light source 13a according to the set value from the controller 30 to output the therapeutic light 80 with the intensity corresponding to the set value. The drive circuit unit 17 controls the output intensity of the therapeutic light 80 to maintain the output intensity constant, based on the output signal of the light-receiving unit 16. With this, the output intensity of the therapeutic light 80 is prevented from fluctuating over time during the treatment in photoimmunotherapy. Note that for the identification light 90, there is no need to control the intensity based on the output signal of the light-receiving unit 16 since precise control of the light intensity is not required.

FIG. 4 also shows the configuration of the light detection unit 40. The light detection unit 40 includes the light input port 41 and a light-receiving unit 42. The light input port 41 is a recess capable of connecting the emission end 22 of the light guide member 20. The light-receiving unit 42 receives the therapeutic light 80 output from the emission end 22 connected to the light input port 41 and outputs a signal corresponding to the received light intensity to the controller 30. The light-receiving unit 42 includes an optical sensor, such as, e.g., a photodiode. Based on the detection signal of the light-receiving unit 42, the controller 30 acquires the intensity of the therapeutic light 80 finally output from the emission end 22 of the light guide member 20, including the light loss, etc., occurred from the first light source 13a to the emission end 22 of the light guide member 20. Based on the acquired intensity of the therapeutic light 80, calibration processing is performed by the controller 30.

### (Therapeutic Light and Identification Light)

Next, the details of the therapeutic light 80 and the identification light 90 will be described. In this embodiment, the light source 10 is configured to emit the therapeutic light 80 of the first wavelength and the identification light 90 of the second wavelength different from the first wavelength, from each of the plurality of light output ports 12.

The therapeutic light 80 is light in the wavelength range in which the fluorescent material of the medicine 102 used in the treatment causes a photochemical reaction in the wavelength region of 600 nm or more and 2,500 nm or less, which is a part of the visible light region to the near-infrared light region. The therapeutic light 80 differs according to the type of fluorescent material of the medicine 102 used in the treatment. As described above, in the case where IR700 is used as the fluorescent material for the medicine 102, the therapeutic light 80 is light with a wavelength peak position of 600 nm or more and 700 nm or less, for example, non-thermal red light (near-infrared light) with a wavelength peak position of about 690 nm.

In this case, the first light source 13a outputs the therapeutic light 80 with a peak wavelength of approximately 690 nm, as the first wavelength. The first light source 13a is capable of outputting, as the therapeutic light 80, laser light of the intensity (energy density) necessary to generate a photochemical reaction in the fluorescent material. Specifically, the first light source 13a outputs the therapeutic light 80 with an intensity equivalent to Class 3 or Class 4 as defined in the International Electrotechnical Standard. Laser light of Class 3 or Class 4 is not permitted to be directly visually observed, so the operators involved in treatment must wear light-shielding glasses 60 (see FIG. 5) for blocking light of a first wavelength so that the therapeutic light 80 is not emitted to the eyes.

The second light source 13b outputs the identification light 90 lower in intensity than the therapeutic light 80. The second light source 13b is controlled to output laser light with intensity deemed to be safe for direct visual observation without wearing light-shielding glasses 60. Specifically, the second light source 13b outputs the identification light 90 with intensity corresponding to, for example, Class 1 as defined in the International Electrotechnical Standard. For this reason, in this embodiment, the identification light 90 emitted from the light guide member 20 can be directly visually observed without wearing light-shielding glasses 60.

Further, in this embodiment, the second wavelength of the identification light 90 is a wavelength outside a first bandwidth of the light-shielding glasses 60 for blocking the light in the first bandwidth including the first wavelength of the therapeutic light 80, and is a wavelength in the range of visible light. In other words, in this embodiment, the identification light 90 is visible light with a wavelength (wavelength whose light transmittance is sufficiently higher than the first bandwidth) that is not blocked by the light-shielding glasses 60. The first bandwidth to which the first wavelength belongs is a wavelength bandwidth in which the red visible light region is partially included. Therefore, the second light source 13b is configured to output light of green (about 490 nm or more and about 550 nm or less), for example, other than red.

As shown in FIG. 5, the lens part 61 of the light-shielding glasses 60 is configured to block the light of the first bandwidth including the first wavelength (approximately 690 nm) of the therapeutic light 80 (i.e., the light transmission of the first wavelength is very small). On the other hand, as the lens part 61 of the light-shielding glasses 60, a lens in which light transmittance of the second wavelength (about 490 nm or more and about 550 nm or less) is sufficiently high is used.

For this reason, when the therapeutic light 80 of the first wavelength (red) is incident on the lens part 61 of the light-shielding glasses 60, it is blocked by the lens part 61. Therefore, it is difficult for the operator wearing the light-shielding glasses 60 to visually recognize the therapeutic light 80. In contrast, when the identification light 90 of the second wavelength (green) is incident on the lens part 61 of the light-shielding glasses 60, the majority of the identification light 90 passes through the lens part 61. Therefore, even a operator wearing the light-shielding glasses 60 can easily visually recognize the identification light 90 through the lens part 61. With this, the identification light 90 in this embodiment can be easily visually recognized even in a state in which the light-shielding glasses 60 are wearing and can be safely visually recognized even in a state in which the light-shielding glasses 60 are removed.

### (Controller)

Next, the controller 30 will be described. As shown in FIG. 6, the controller 30 is configured by a computer including a processor 31 and a storage unit 32.

The processor 31 is configured, for example, by a CPU (Central Processing Unit), an FPGA (Field-Programmable Gate Array), or the like. The storage unit 32 includes, for example, a volatile memory and a nonvolatile memory, such as, e.g., a flash memory and a hard disk drive. The storage unit 32 stores the program 32a. The computer functions as the controller 30 of the light irradiation device 100 by the processor 31 executing the program 32a stored in the storage unit 32. Further, various setting information 32b for light irradiation is stored in the storage unit 32.

The controller 30 includes, as functional blocks, a light source control unit 31a, a calibration processing unit 31b, and a display control unit 31c. The functional block means a group of functions of information processing realized by the processor 31 provided by the controller 30 executing the program 32a. Each of these functional blocks may be configured by separate hardware (processor).

### (Light Source Control Unit)

The light source control unit 31a controls the start of light output and the stop of light output by the light source 10. The light source control unit 31a controls each light source unit 11 (see FIG. 1) of the light source 10 to output the therapeutic light 80 of the set intensity from the set light output port 12 during the set time based on the setting information 32b stored in the storage unit 32.

When the light source control unit 31a receives an operation input to the second operation unit 53 while the output of the therapeutic light 80 is being stopped, the light source control unit 31a starts the output of the therapeutic light 80. The light source control unit 31a stops the output of the therapeutic light 80 when the set time has elapsed or when it receives an operation input to the second operation unit 53 before the elapse of the set time during the therapeutic light 80 is being output.

### (Calibration Processing Unit)

The calibration processing unit 31b performs calibration processing based on the detection result of the light detection unit 40 (see FIG. 4). The calibration processing unit 31b is configured to determine whether the calibration processing has been successfully completed based on the comparison between the detection result of the light detection unit 40 and the set value of the intensity of the therapeutic light 80.

The calibration processing is performed for each of the plurality of light output ports 12 (i.e., the plurality of light source units 11) sequentially. First, as shown in FIG. 4, the emission end 22 of the light guide member 20 connected to one light output port 12, which is the target of the calibration processing, is connected by the operator to the light input port 41 of the light detection unit 40. The calibration processing unit 31b causes the therapeutic light 80 to be output from one light output port 12 subject to calibration processing. The therapeutic light 80 that has passed through the light guide member 20 and has been output from the emission end 22 is detected by the light-receiving unit 42 of the light detection unit 40. The calibration processing unit 31b acquires a signal corresponding to the light-receiving intensity from the light detection unit 40.

The calibration processing unit 31b acquires the detected value of the intensity of the therapeutic light 80 at the emission end 22 of the light guide member 20 connected to the light input port 41, based on the detection result (signal corresponding to the received light intensity) of the light detection unit 40. The calibration processing unit 31b compares the detection unit and the intensity set value to determine whether the detection value is within an acceptable range for the intensity set value. The calibration processing unit 31b adjusts the setting of the drive level of the first light source 13a by the drive circuit unit 17 of the light source unit 11 so that the detection value falls within the acceptable range when the detection value is not within the acceptable range. As a result, the intensity of the therapeutic light 80 output from the light guide member 20 connected to the one light output port 12 subject to the calibration processing is adjusted to match the intensity setting value of the setting information 32b within an acceptable range.

The calibration processing unit 31b determines that the calibration is normally performed when the detection unit falls within the allowable range. As described above, the controller 30 (calibration processing unit 31b) determines whether the calibration processing has been successfully completed based on a comparison between the detection result of the light detection unit 40 and the set value of the emission intensity of the therapeutic light 80. In this embodiment, the controller 30 (calibration processing unit 31b) is configured to control the light source 10 to emit the identification light 90 (see FIG. 2) when the calibration processing has been successfully completed.

When the calibration processing for one light output port 12 is completed, the light guide member 20 connected to the light input port 41 of the light detection unit 40 is removed by the operator, and the light guide member 20 connected to the light output port 12 to be calibrated next is connected to the light input port 41. The controller 30 (calibration processing unit 31b) performs calibration processing for each of the plurality of light output ports 12 sequentially.

### (Display Control Unit)

The display control unit 31c controls the screen display of the display unit 51. The display control unit 31c causes the operation reception screen 70 to be displayed on the display unit 51, as shown in FIG. 7. The display control unit 31c is configured to receive various operations and setting inputs via the first operation unit 52 (see FIG. 1) on the operation reception screen 70.

### (Operation Reception Screen)

In the example shown in FIG. 7, the operation reception screen 70 includes a setting information display region 71, a treatment procedure display region 72, a first identification operation unit 73a, and a second identification operation unit 73b.

### (Setting Information Display Region)

The setting information display region 71 is a region for displaying setting information (setting information 32b) concerning the emission of the therapeutic light 80 by the light source 10. The setting information display region 71 is provided one for each of the plurality of light output ports 12 ("PORT 1" to "PORT 4") of the light source 10. In other words, there is a plurality (four) of setting information display regions 71 corresponding to the plurality (four) light output ports 12, respectively, one-to-one.

Each setting information display region 71 includes a type display unit 71a for the light guide member 20, an optical specification display unit 71b for the light guide member 20, an irradiation energy display unit 71c, and an irradiation time display unit 71d. The display control unit 31c performs information display of the setting information display region 71 according to the setting information 32b stored in the storage unit 32. The setting information 32b is set by the operator according to the treatment condition by photoimmunotherapy.

The type display unit 71a displays the type of the light guide member 20 according to the type (structure) of the light diffusion member constituting the emission end 22. The type display unit 71a displays the type of the light guide member 20 in the form of a pictogram (icon). The pictogram of the various type display units 71a in FIG. 7 indicates that light (represented by arc-shaped lines) is emitted from a rod-shaped light diffusing member to the surrounding area and indicates that a circumferential emission type light guide member 20 (see FIG. 3(B)) is set. In the case where a forward emission type light guide member 20 (see FIG. 3(A)) is set in the setting information 32b, a pictogram (not shown) indicating that the light guide member is a forward emission type is displayed.

The optical specification display unit 71b displays the optical specification for the irradiation of the therapeutic light 80 in the light guide member 20 displayed on the type display unit 71a. The optical specification display unit 71b displays pre-specified information (parameters) considered to be important when emitting the therapeutic light 80 to the treatment target site 101a, among other optical specifications. The example in FIG. 7 shows information on the range of the therapeutic light 80 emitted at the emission end 22 (light diffusion member) of the circumferential emission type light guide member 20. The range where the therapeutic light 80 is emitted is represented by the distance 25 (see FIG. 3((B)) from the tip of the rod-shaped light-diffusing member. In FIG. 7, it is displayed as "2 cm," indicating that the therapeutic light 80 is emitted from a portion of 2 cm from the tip of the light-diffusing member. In the case where a forward emission type light guide member 20 (see FIG. 3(A)) is displayed on the type display unit 71a, information on the irradiation angle 26 or the like can be displayed.

The irradiation energy display unit 71c displays the set value of the total energy of the therapeutic light 80 emitted from the light guide member 20 to the treatment target site 101a during treatment. Further, the irradiation time display unit 71d displays the set value of the irradiation time of the therapeutic light 80 emitted from the light guide member 20 to the treatment target site 101a during treatment. The total energy amount is expressed by the energy amount per unit time (J/cm), and the irradiation time is expressed by the total value (sec) of the time during which the therapeutic light 80 is emitted.

By the optical specification display unit 71b, the irradiation energy display unit 71c, and the irradiation time display unit 71d, the example in FIG. 7 shows that the condition is set that when the therapeutic light 80 is irradiated from a range of 2 cm from the tip of the light guide member 20 for 250 seconds, a total of 100 J/cm of the therapeutic light 80 will be irradiated to the treatment target site 101a. The controller 30 sets the light intensity of the therapeutic light 80 emitted from the light output port 12 to satisfy this condition.

### (Treatment Procedure Display Region)

The treatment procedure display region 72 displays the treatment procedure (protocol) using the light irradiation device 100. The operator can prepare the treatment and perform the treatment procedure by referring to the contents of the display in the treatment procedure display region 72. In the example in FIG. 7, as Step 1, the preparation (Standby), such as, e.g., a connection operation of the light guide member 20 and an input of setting information 32b, is displayed. As Step 2, the calibration processing (Calibrate) of each light output port 12 is shown. As Step 3, the output (Treatment) of the therapeutic light 80 is shown. The output of the therapeutic light 80 may be performed in a plurality of separated manners. Further, in the case where treatment is performed by replacing the light guide member 20 (by replacing the circumferential emission type with the forward emission type), the calibration processing and the output of the therapeutic light 80 may be sometimes performed in a plurality of separated manner.

### (First Identification Operation Unit and Second Identification Operation Unit)

The first identification operation unit 73a and the second identification operation unit 73b are buttons each for receiving an operation input to emit the identification light 90 (see FIG. 2) from the light output port 12. The first identification operation unit 73a and the second identification operation unit 73b each are a GUI (Graphical User Interface) displayed on the operation reception screen 70.

In the example of FIG. 7, four first identification operation units 73a are provided on the operation reception screen 70 according to the four light output ports 12. In other words, the first identification operation unit 73a is a "Check" button provided for each of the four setting information display regions 71 (PORT 1 to PORT 4) corresponding to the light output ports 12, respectively.

In this embodiment, the first operation unit 52, which is a touch panel, detects the input operation (touch operation) to the first identification operation unit 73a and outputs a signal corresponding to the operation input to the controller 30. The controller 30 (light source control unit 31a) controls the light source 10 to emit the identification light 90 from the light output port 12 corresponding to the first identification operation unit 73a that received the operation input out of the plurality of first identification operation units 73a.

As described above, in this embodiment, the light irradiation device 100 is provided with a plurality of first identification operation units 73a, which are provided corresponding to the plurality of light output ports 12 to receive an operation input to instruct the emission of the identification light 90 for each of the light output ports 12. Therefore, by selectively operating the plurality of first identification operation units 73a, the operator can cause the identification light 90 to be output from one light guide member 20 connected to the designated light output port 12 out of the four light output ports 12.

On the other hand, the second identification operation unit 73b is a button for outputting the identification light 90 from the plurality of light output ports 12 at the same time.

In the example of FIG. 7, one second identification operation unit 73b is provided on the operation reception screen 70 for the four light output ports 12. The second identification operation unit 73b is a large "All Check" button provided across the four setting information display regions 71 each provided for one port. As described above, in this embodiment, the light irradiation device 100 is equipped with the second identification operation unit 73b, which is provided one for the plurality of light output ports 12 and receives an operation input that instructs the emission of the identification light 90 from the plurality of light output ports 12.

In this embodiment, the first operation unit 52, which is a touch panel, detects the input operation (touch operation) to the second identification operation unit 73b and outputs a signal corresponding to the operation input to the controller 30.

Here, in this embodiment, the light source 10 is configured to simultaneously emit the identification light 90 different from each other in the pattern of lighting-on and lighting-off in unit time from each of the plurality of light output ports 12. The controller 30 controls the light source 10 to emit the identification light 90 different in pattern from each other from each of the plurality of light output ports 12 in response to the operation input to the second identification operation unit 73b. Note that "Lighting-on" and "Lighting-off" mean the irradiation of the identification light 90 and the irradiation stop of the identification light 90, respectively.

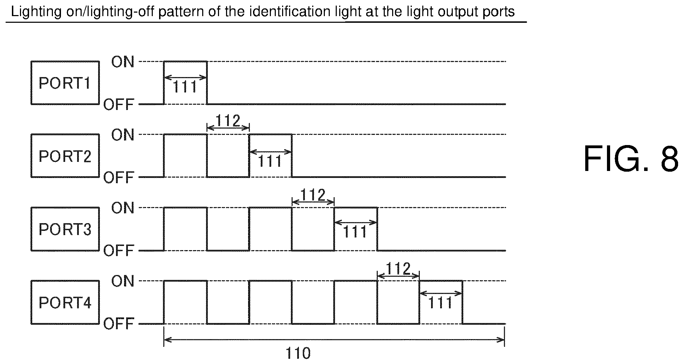

One example of the patterns of lighting-on and lighting-off in unit time is shown in FIG. 8. FIG. 8 shows a timing chart in which the horizontal axis represents time and the switching timings of lighting-on (ON) and lighting-off (OFF) at the four light output ports 12 (PORT 1 to PORT 4) are shown side by side.

PORT 1 blinks, during the unit time 110, in a pattern in which lighting-on in the lighting-on time 111 is performed once, and lighting-off is performed during the time other than the lighting-on time 111. PORT 2 blinks, during the unit time 110, in a pattern in which lighting-on in the lighting-on time 111 is performed twice in the lighting-on intervals 112, and lighting-off is performed during the time other than the lighting-on time 111. PORT 3 blinks, during the unit time 110, in a pattern in which lighting-on in the lighting-on time 111 is performed three times in the lighting-on intervals 112, and lighting-off is performed during the time other than the lighting-on time 111. PORT 4 blinks, during the unit time 110, in a pattern in which lighting-on in the lighting-on time 111 is performed four times in the lighting-on intervals 112, and lighting-off is performed during the time other than the lighting-on time 111. Note that the lighting-on time 111 is a length of time during which the lighting-on state continues. The lighting-on interval 112 is a length of time during which the lighting-off state continues between a lighting-on state and the subsequent lighting-on state. Here, all of PORTs blink (light on and off), but for any PORT, the pattern may be a continuous lighting-on state instead of a blinking pattern.

As described above, in the case where the identification light 90 is turned on via the four light guide members 20 in response to the operation input to the second identification operation unit 73b, the number of times of lighting-on of the identification light 90 in the unit time 110 differs for each of the light output ports 12. As a result, the operator can determine which light guide member 20 is connected to which light output port 12 by the frequency of the blinks of the emission end 22 of the light guide member 20. In the example in FIG. 8, the identification light 90 is turned on by the same number of times as the port numbers of PORT 1 to PORT 4 during the unit time 110. Therefore, it is easy to determine the port number to which the light guide member 20 is connected by the number of lighting times of the emission end 22.

Note that while the identification light 90 is emitted from each of the four light guide members 20 in response to the operation input to the second identification operation unit 73b, the display control unit 31c may control the display unit 51 such that each of the four setting information display regions 71 corresponding to the respective light output ports 12 blinks in the same pattern as the pattern of lighting-on/lighting-off of the identification light 90. In other words, each of the setting information display regions 71 of PORT 1 to PORT 4 shown in FIG. 7 may be blinked in the pattern of PORT 1 to PORT 4 shown in FIG. 8.

In this embodiment, the controller 30 (light source control unit 31a) controls the light source 10 to output the identification light 90 only while the first identification operation unit 73a or the second identification operation unit 73b is being operated. Therefore, when the operator touches the first identification operation unit 73a or the second identification operation unit 73b, the output of the identification light 90 is initiated, and when the operator removes the finger from the touch panel to release the touch, the output of the identification light 90 is terminated.

### (Control Processing of Light Irradiation Device)

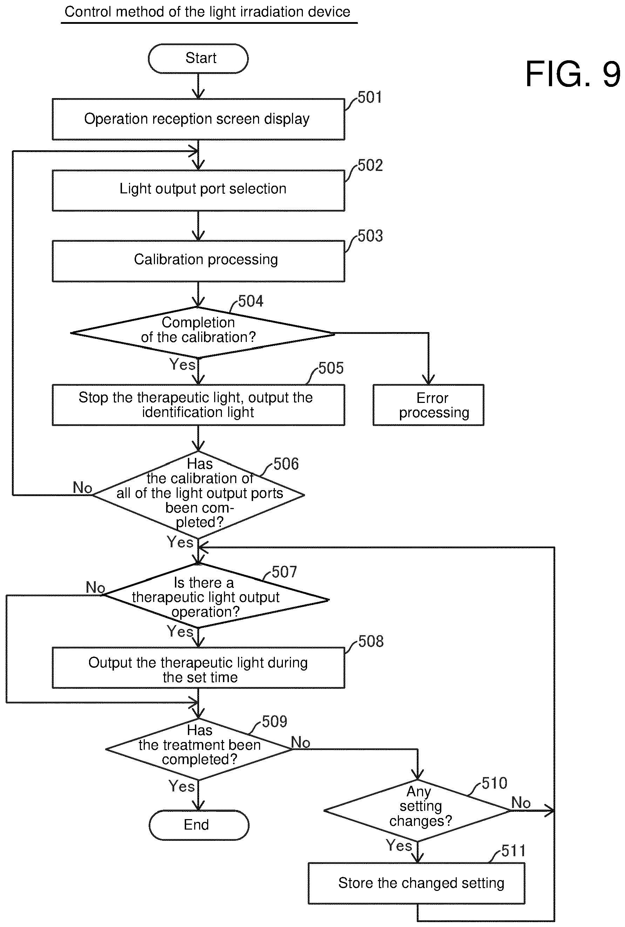

Next, the control processing of the light irradiation device 100 will be described with reference to FIG. 9. The control processing of the light irradiation device 100 includes a control method for the light irradiation device according to this embodiment. The control processing of the light irradiation device 100 is performed by the controller 30.

In Step 501 of FIG. 9, the controller 30 (display control unit 31c) performs the control to display the operation reception screen 70 on the display unit 51.

In Step 502, the controller 30 (display control unit 31c) selects one light output port 12 to be subjected to calibration processing. The selection order of the light output ports 12 may be, for example, in order of decreasing the port number, or it may be specified by the operator.

The operator connects the emission end 22 of one light guide member 20 connected to the selected light output port 12 to the light input port 41. The operator operates the first identification operation unit 73a or the second identification operation unit 73b on the operation reception screen 70 (see FIG. 7) to cause the identification light 90 to be emitted from the light guide member 20. For example, in the case where the light output port 12 to be subject to calibration processing is PORT 1, the operator operates the first identification operation unit 73a of PORT 1. With this, the identification light 90 (see FIG. 2) is emitted from the light guide member 20 connected to PORT 1, and therefore, the operator can easily distinguish the light guide member 20 connected to PORT 1 from the other light guide members 20. The control regarding the emission of the identification light 90 will be described later.

In Step 503, the controller 30 (calibration processing unit 31b) performs calibration processing for the light output port 12 to be subjected to calibration processing. The calibration processing unit 31B causes the therapeutic light 80 to be emitted from the target light output port 12, as described above, to acquire the detection result from the light detection unit 40. The calibration processing unit 31b adjusts such that the detection result of the light detection unit 40 (the emission intensity of the therapeutic light 80 from the emission end 22) matches the intensity set value within an acceptable range, based on the comparison between the detection result of the light detection unit 40 and the set value of the emission intensity of the therapeutic light 80.

In Step 504, the controller 30 (calibration processing unit 31b) determines whether the calibration processing has been successfully completed, based on the comparison between the detection result of the light detection unit 40 and the intensity set value of the therapeutic light 80. Even in the case where the detection result of the light detection unit 40 does not match the intensity set value within an acceptable range even after the calibration processing is performed, the controller 30 (calibration processing unit 31b) discontinues the subsequent processing and performs predetermined error processing. In the case where the detection result of the light detection unit 40 matches the intensity set value within an acceptable range, the controller 30 (calibration processing unit 31b) determines that the calibration processing has been successfully completed and advances the processing to Step 505.

In Step 505, the controller 30 (calibration processing unit 31b) stops the output of the therapeutic light 80 from the light output port 12 for which the calibration processing has been completed and controls the light source 10 to emit the identification light 90. In other words, when the calibration processing for any of the light output ports 12 has been completed, the controller 30 (calibration processing unit 31b) informs the operator of the completion of the calibration processing by emitting the identification light 90 from the light output port 12. The emission of the identification light 90 is stopped after elapsing a predetermined period of time.

In Step 506, the controller 30 (calibration processing unit 31b) determines whether the calibration processing has been completed for all of the light output ports 12 to be subjected to calibration processing. Further, in the case where there are light output ports 12 for which calibration processing has not yet been performed, the controller 30 (calibration processing unit 3 1b) returns the processing to Step 502 and selects the light output ports 12 for which calibration processing has not yet been performed as a calibration processing target. By repeating Steps 502 to 506, the calibration processing is sequentially performed on the plurality of light output ports 12 one by one. When calibration processing has been completed for all of the light output ports 12, the controller 30 advances the processing to Step 507.

In Step 507, the controller 30 (light source control unit 31a) determines whether an operation input instructing the output of the therapeutic light 80 is received. The controller 30 (light source control unit 31a) receives an operation input to instruct the output of the therapeutic light 80 by the second operation unit 53 (see FIG. 1).

Before performing an operation input, the operator positions the plurality of light guide members 20 connected to the plurality of light output ports 12, respectively, at predetermined positions for irradiating the treatment target site 101a of the patient 101 with the therapeutic light 80. In the case of the circumferential emission type light guide member 20, as described above, the emission end 22 of the light guide member 20 is inserted to a predetermined position in the body of the patient 101. After completion of the irradiation preparation, the operator performs an operation input to the second operation unit 53. When an operation input instructing the output of the therapeutic light 80 is received (operation to the second operation unit 53 is received), the controller 30 (light source control unit 31a) advances the processing to Step 508. In the case where no operation input has been received, the controller 30 (light source control unit 31a) advances the processing to Step 509.

In Step 508, the controller 30 (light source control unit 31a) controls the light source 10 to output the therapeutic light 80 from each of the plurality of light output ports 12. With this, the therapeutic light 80 is emitted from the emission end 22 of the light guide member 20 connected to the light output port 12 to the treatment target site 101a. The controller 30 (light source control unit 31a) outputs the therapeutic light 80 during the set irradiation time (see FIG. 7) based on the setting information 32b.

In Step 509, the controller 30 (light source control unit 31a) determines whether to terminate the treatment. The condition for terminating one treatment (irradiation of the therapeutic light 80) is, for example, that the total irradiation time of the therapeutic light 80 has reached a set irradiation time. Further, the controller 30 (light source control unit 31a) determines to terminate the treatment when, for example, it receives an input operation input indicating the termination of the treatment in the GUI displayed on the display unit 51. In that case, the control processing of the light irradiation device 100 is completed.

In the case where the conditions for terminating the treatment have not been met, the controller 30 determines in Step 510 whether the setting information 32b has been changed.

The operator operates the first operation unit 52 to display the setting screen on the display unit 51 from the operation reception screen 70, which is not shown in the figure, and operates to change the setting information 32b for each light output port 12 on the setting screen. With this, it is possible to change the type of the light guide member 20 and the setting information 32b, such as the irradiation time and the total energy, for each light output port 12. In this case, there may be a case in which the operator needs to check which of the plurality of light guide members 20 out of the plurality of light guide members 20 is connected to the light output port 12 that he/she intends to change the setting.

At this time, the operator can cause the identification light 90 to be emitted from the light guide member 20 by operating the first identification operation unit 73a or the second identification operation unit 73b. With this, the identification light 90 is emitted from the light guide member 20 connected to the light output port 12 for which the setting is to be changed, and therefore, it is possible to easily distinguish the light guide member 20 connected to the light output port 12 for which the operator intends to change the setting, from other light guide members 20.

In the case where no change in the setting information 32b is received, the controller 30 returns the processing to Step S507. In the case where the change of the setting information 32b is received, the controller 30 stores the changed setting information 32b in the storage unit 32 in Step 511, and then returns the processing to Step S507. With this, for example, the irradiation conditions of the therapeutic light 80 are changed, and a second irradiation of the therapeutic light 80 is performed. Note that depending on the contents of the changed setting information 32b, the processing may be returned to Step 502 for calibration processing.

When all of the scheduled treatments have been completed, the controller 30 determines that the treatment has been completed in Step 509, and terminates the control processing of the light irradiation device 100.

### (Output Control of Identification Light)

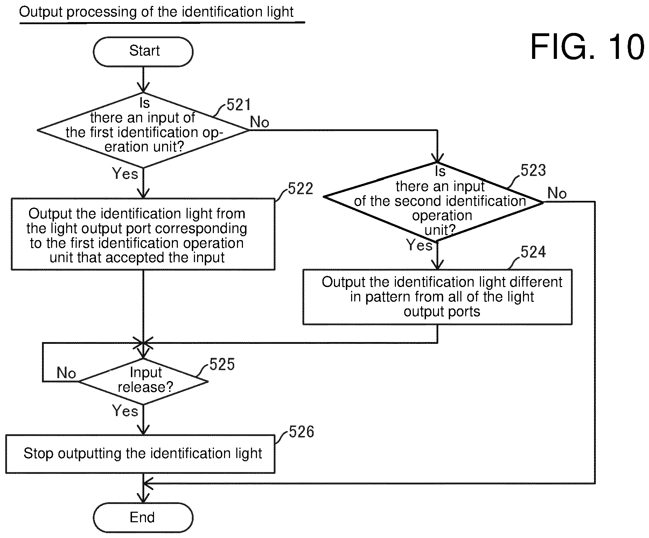

Referring to FIG. 10, the output control of the identification light 90 will be described. The output control of the identification light 90 is performed by the controller 30. The output control of the identification light 90 is processing that is executed in parallel with the execution of the control processing of the light irradiation device 100 shown in FIG. 9.

In Step 521, the controller 30 (light source control unit 31a) determines whether an input operation to any of the first identification operation units 73a has been received via the first operation unit 52.

When an input operation to any of the first identification operation units 73a is received, the controller 30 (light source control unit 31a) controls the light source 10 to output the identification light 90 from the light output port 12 corresponding to the first identification operation unit 73a for which the input operation was received, in Step 522. For example, when an operation input is received for the first identification operation unit 73a corresponding to the setting information display region 71 of PORT 1 shown in FIG. 7, the controller 30 causes the identification light 90 to be output from the light output port 12 of PORT 1. Note that in the case where operation inputs are made simultaneously to the plurality of first identification operation units 73a, the controller 30 (light source control unit 31a) causes the identification light 90 to be output simultaneously from the corresponding light output ports 12.

In the case where no input operation to the first identification operation unit 73a has been received in Step 521, the controller 30 (light source control unit 31a) advances the processing to Step 523. In Step 523, the controller 30 (light source control unit 31a) determines whether an input operation to the second identification operation unit 73b has been received via the first operation unit 52.

In the case where an input operation to the second identification operation unit 73b has been received, the controller 30 (light source control unit 31a) controls the light source 10 to output the identification light 90 with different lighting-on and lighting-off patterns from all the light output ports 12 in Step 524. For example, the controller 30 (light source control unit 31a) causes each of the light output ports 12 of PORT 1 to PORT 4 to output the identification light 90 in the pattern shown in FIG. 8. In Step 523, in the case where no input operation input to the second identification operation unit 73b has been received, the processing is terminated.

In the case where the identification light 90 is output in Step 522 or Step 524, the controller 30 (light source control unit 31a) determines in Step 525 whether the operation input to the first identification operation unit 73a or the second identification operation unit 73b is released. In the case of this embodiment in which the first operation unit 52 is a touch panel, it is determined whether the touch operation is released. In the case where the operation input is not released, the controller 30 (light source control unit 31a) continuously outputs the identification light 90.

When the operation input is released, the controller 30 (light source control unit 31a) stops outputting the identification light 90 in Step 526. Therefore, in this embodiment, the identification light 90 is output only while the operation input to the first identification operation unit 73a or the second identification operation unit 73b continues.

When the output of the identification light 90 is stopped in Step 526, the controller 30 (light source control unit 31a) terminates the processing. The controller 30 (light source control unit 31a) always receives an operation input to the first identification operation unit 73a or the second identification operation unit 73b by repeating the processing of Steps 521 to 526 shown in FIG. 10. This allows the operator to output the identification light 90 only at the desired timing for the desired amount of time. By this processing, the identification light 90 will be output at the timing when the operator operates the first identification operation unit 73a or the second identification operation unit 73b in Step 502 or Step 510 of FIG. 9. Even at timings other than Step 502 or Step 510, the controller 30 (light source control unit 31a) performs the control to output the identification light 90 in parallel with each processing in FIG. 9, when the first identification operation unit 73a or the second identification operation unit 73b is operated.

As described above, the control method for the light irradiation device 100 is a control method for a light irradiation device 100 for irradiating a treatment target site 101a of a subject (cancer patient 101) to which a medicine 102 containing a photosensitive substance has been administered, with therapeutic light 80. The control method includes: a step (522, 524) of causing the plurality of light guide members 20 connected to the plurality of light output ports 12, respectively, to light up in an identifiable manner by emitting the identification light 90 lower in intensity than the therapeutic light 80 from each of the plurality of light output ports 12 to which the plurality of light guide members 20 is connected, respectively; and a step (508) of emitting the therapeutic light 80 from each of the plurality of light output ports 12.

### (Effects of This Embodiment)

In this embodiment, the following effects can be obtained.

That is, as described above, the light irradiation device 100 of this embodiment is a light irradiation device 100 for irradiating a treatment target site 101a of a subject (cancer patient 101) to which a medicine 102 containing a photosensitive substance has been administered with therapeutic light 80. The light irradiation device is provided with a light source 10 including a plurality of light output ports 12 and configured to emit the therapeutic light 80 and the identification light 90 lower in intensity than the therapeutic light 80, from each of the plurality of light output ports 12, a plurality of light guide members 20 having an incident end 21 to be connected to any one of the plurality of light output ports 12 of the light source 10 and an emission end 22 for emitting the light incident from the incident end 21, and a controller 30 for controlling the light source 10. The controller 30 is configured to perform control to cause a plurality of light guide members 20 connected to the plurality of light output ports 12 to be light up in an identifiable manner by emitting an identification light 90 from each of the plurality of light output ports 12.

Further, as described above, the control method for the light irradiation device 100 is a control method for a light irradiation device 100 for irradiating a treatment target site 101a of a subject (cancer patient 101) to which a medicine 102 containing a photosensitive substance has been administered, with therapeutic light 80. The control method includes: a step of causing the plurality of light guide members 20 connected to the plurality of light output ports 12, respectively, to be light up in an identifiable manner by emitting the identification light 90 lower in intensity than the therapeutic light 80 from each of the plurality of light output ports 12 to which the plurality of light guide members 20 are connected; and a step of emitting the therapeutic light 80 from each of the plurality of light output ports 12.

With the above-described configuration, by emitting the identification light 90 lower in intensity than the therapeutic light 80 from each of the plurality of light output ports 12, a control to perform lighting-on of the plurality of light guide members 20 connected to the plurality of light output ports 12, respectively, in an identifiable manner is performed. For example, the identification light 90 is caused to be emitted separately from the plurality of light output ports 12 one by one, or the identification light 90 with different lighting patterns (e.g., blinking speed) is caused to be emitted from the plurality of light output ports 12 at the same time. With this, it is possible to confirm whether the identification light 90 from an individual light output port 12 is emitted from which of the plurality of light guide members 20. At this time, the identification light 90 lower in intensity than the therapeutic light 80 is emitted from the light guide member, so that the identification light 90 can be confirmed without the use of light-shielding glasses 60. As a result, even in the case where a plurality of light guide members 20 remain entangled, it is possible to easily identify which light guide member 20 is connected to which light output port 12 by the identification light 90. Further, with this, it becomes possible to shorten the preparation time required before initiating treatment.

Further, in the above-described first embodiment, the following further effects can be obtained by configuring as follows.

Specifically, in the light irradiation device 100 of this embodiment, the light source 10 is configured to emit the therapeutic light 80 of a first wavelength and the identification light 90 of a second wavelength different from the first wavelength from each of the plurality of light output ports 12. By configuring as described above, it is possible to easily distinguish the identification light 90 from the therapeutic light 80 by the identification light 90 of a different second wavelength (i.e., a color different from that of the therapeutic light 80).

Further, in the light irradiation device 100 of this embodiment, the second wavelength of the identification light 90 is a wavelength outside the first bandwidth of the light-shielding glasses 60 that block the first bandwidth of the light and is a wavelength within the range of visible light. By configuring as described above, it is possible to visually recognize the identification light 90 through the light-shielding glasses 60 while wearing the light-shielding glasses 60 that block the therapeutic light 80. For this reason, it is unnecessary to remove the light-shielding glasses 60 each time the connection of the light guide member 20 is checked by the identification light 90, which can improve the workability related to therapeutic light.

Further, in this embodiment of the light irradiation device 100, the light source 10 includes the first light source 13a for generating the therapeutic light 80 and the second light source 13b for generating the identification light 90 and is configured to emit the therapeutic light 80 and the identification light 90 independently from the plurality of light output ports 12. By configuring as described above, the first light source 13a dedicated to the therapeutic light 80 and the second light source 13b dedicated to the identification light 90 are provided, so that the therapeutic light 80 of the first wavelength and the identification light 90 of the second wavelength can be emitted without using, for example, a special wavelength-tunable laser light source. Therefore, the light source 10 that emits the therapeutic light 80 and the identification light 90 can be easily configured.

Further, in the light irradiation device 100 of this embodiment, the light source 10 is configured to be able to individually switch the emission and the emission stop of the identification light 90 from each of the plurality of light output ports 12. By configuring as described above, it is possible to selectively emit the identification light 90 from the plurality of light output ports 12 one at a time. Therefore, it is possible to very easily identify which light guide member 20 is connected to which light output port 12.

Further, the light irradiation device 100 of this embodiment is further provided with a plurality of first identification operation units 73a provided corresponding to the plurality of light output ports 12 and configured to receive an operation input instructing the emission of the identification light 90 for each of the light output ports 12. The controller 30 controls the light source 10 to emit the identification light 90 from the light output port 12 corresponding to the first identification operation unit 73a for which the operation input is received out of the plurality of first identification operation units 73a. By configuring as described above, the operator can individually identify the light guide member 20 connected to the light output port 12 to be checked by simply operating the first identification operation unit 73a corresponding to the light output port 12 to be checked. For this reason, for example, in the calibration processing, it becomes very easy to identify one light guide member 20 connected to the light output port 12 to be subjected to the calibration processing from the plurality of a light guide member 20.

Further, in the light irradiation device 100 of this embodiment, the light source 10 is configured to simultaneously emit the identification light 90 different in pattern of lighting-on and lighting-off in unit time 110 from each of the plurality of light output ports 12. By configuring as described above, it is possible to collectively identify the interconnection relation between the plurality of light guide members 20 and the plurality of light output ports 12, based on the difference in the patterns of the identification light 90 emitted from each of the plurality of light guide members 20.

Further, in the light irradiation device 100 of this embodiment, it is further provided with a second identification operation unit 73b, one being provided for each of the plurality of light output ports 12, the second identification operation unit 73b being configured to receive an operation input indicating the emission of the identification light 90 from the plurality of light output ports 12. The controller 30 controls the light source 10 to emit the identification light 90 different in pattern from each of the plurality of light output ports 12 in response to the operation input to the second identification operation unit 73b. By configuring as described above, the operator can collectively identify the interconnection relation between the plurality of light output ports 12 and the plurality of light guide members 20 by simply operating the second identification operation unit 73b. For this reason, it becomes very easy to check the connection in the case where the user wants to know the connection destination (the number of the light output port 12) for all of the light guide members 20.

Further, the light irradiation device 100 of this embodiment is further provided with the light detection unit 40 that accepts the emission end 22 of any one of the plurality of light guide members 20 to detect the light output from the emission end 22. The controller 30 is configured to perform calibration processing for adjusting the emission intensity of the therapeutic light 80 at each of the plurality of light output ports 12, based on the detection result of the light detection unit 40. By configuring as described above, it becomes necessary to connect the emission end 22 of the light guide member 20 connected to the light output port 12 to be subjected to calibration processing to the light detection unit 40. However, this makes it extremely easy to identify the light guide member 20 connected to the light output port 12 to be subjected to calibration processing by causing the identification light 90 to be emitted from the light guide member 20. Therefore, it is possible to markedly improve the work efficiency of the calibration processing for each of the plurality of light output ports 12, and therefore, the preparation time required before starting the treatment can be effectively shortened.

Further, in the light irradiation device 100 of this embodiment, the controller 30 determines whether the calibration processing has been successfully completed based on the comparison between the detection result of the light detection unit 40 and the set value of the output intensity of the therapeutic light 80, and when the calibration processing has been successfully completed, the controller performs the control to cause the light source 10 to emit the identification light 90. By configuring as described above, the operator can easily recognize that the calibration processing has been successfully completed by the identification light 90 emitted from the light guide member 20. In addition, since the light guide members 20 for which calibration processing has been completed and the light guide members 20 for which calibration processing will be performed can be identified, it is possible to prevent the operator from selecting a wrong light guide member 20 to be subjected to calibration processing from a plurality of light guide members 20.

### <Modifications>

Note that the embodiments disclosed here should be considered illustrative and not restrictive in all respects. It should be noted that the scope of the present invention is indicated by claims and is intended to include all modifications (modified examples) within the meaning and scope of the claims and equivalents.

### (First Modification)

For example, in the above-described embodiment, an example is shown in which the light source 10 includes the first light source 13a for generating the therapeutic light 80 and the second light source 13b for generating the identification light 90, but the present invention is not limited thereto. In the present invention, the light source for generating the therapeutic light 80 and the light source for generating the identification light 90 may be the same light source.

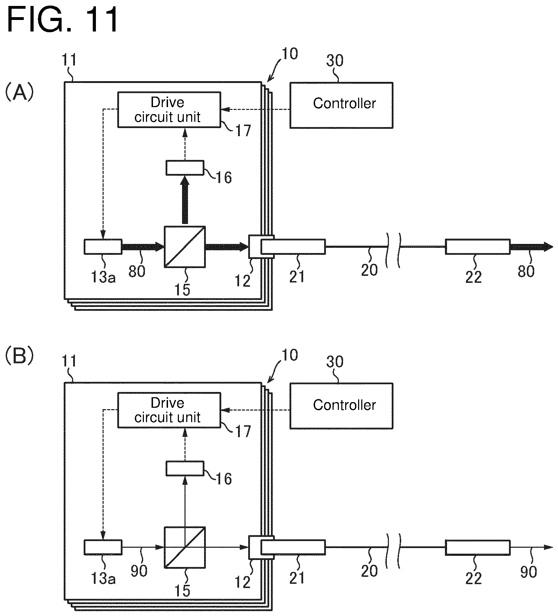

For example, in FIG. 11, each light source unit 11 of the light source 10 includes a single first light source 13a. The light source unit 11 is not provided with a dedicated light source (second light source 13b) for generating identification light 90, and the first light source 13a generates the therapeutic light 80 and the identification light 90.

Specifically, in this modification, as shown in FIG. 11(A), the drive circuit unit 17 causes the first light source 13a to output the therapeutic light 80 of the first wavelength, based on the signal from the controller 30. The therapeutic light 80 is laser light with intensity equivalent to Class 3 or Class 4. As shown in FIG. 11(B), based on the signal from the controller 30, the drive circuit unit 17 causes the first light source 13a to output the identification light 90 of a first wavelength. The identification light 90 is light of the first wavelength generated by the first light source 13a and is light lower in intensity than the therapeutic light 80. For example, the identification light 90 is laser light with intensity corresponding to Class 1. In other words, the light source 10 uses the light emitted from the same first light source 13a as the identification light 90 by reducing the output intensity of the light at the first light source 13a for generating the therapeutic light 80 to the level of class 1. Note that in FIG. 11, the difference in the intensity of the therapeutic light 80 and the identification light 90 are represented by arrows with different thicknesses.

In this modification, the therapeutic light 80 and the identification light 90 are light of the same wavelength (first wavelength). In this case, the identification light 90 is light of a wavelength belonging to the first bandwidth that is blocked by the light-shielding glasses 60, so the operator cannot visually recognize the identification light 90 while wearing the light-shielding glasses 60. However, because the identification light 90 is laser light with intensity equivalent to Class 1, the identification light 90 can be safely and reliably visually recognized in a state in which the operator removed his or her light-shielding glasses 60. Therefore, in this modification, if necessary, the operator can easily identify which light guide members 20 is connected to which light output port 12 by removing the light-shielding glasses 60 to check the identification light 90.

Therefore, by configuring the light irradiation device 100 as in this modification, by making the identification light 90 sufficiently lower intensity than the therapeutic light 80, it is possible to confirm the identification light 90 by removing the light-shielding glasses 60. In this case, the identification light 90 can be generated using the first light source 13a for the therapeutic light 80 without providing a separate light source for the identification light 90, so it is possible to simplify the configuration of the device to enable the emission of the identification light 90 separately from the therapeutic light 80.

Alternatively, the light source 10 may be equipped with a wavelength tunable laser device as a light source capable of changing the wavelength of the outputting light between a first wavelength and a second wavelength different from the first wavelength. In this case, it is possible to output the therapeutic light 80 of the first wavelength and the identification light in the second wavelength bandwidth high in transmittance of the light-shielding glasses 60, by a single light source.

### [Other Modification)]

In the above-described embodiment, an example is shown in which the light source 10 is configured to emit the therapeutic light 80 and the identification light 90 independently from the plurality of light output ports 12, but the present invention is not limited thereto. In the present invention, the light source 10 need not be capable of emitting the therapeutic light 80 and the identification light 90 independently from the plurality of light output ports 12. For example, the light source 10 may be configured to emit the therapeutic light 80 from all of the light output ports 12 simultaneously and the identification light 90 from all of the light output ports 12 simultaneously. In this case, the light source 10 is not required to have a plurality of light source units 11. In other words, light may be distributed from one light source unit 11 to a plurality of light output ports 12.

Further, in the above-described embodiment, an example is shown in which the light source 10 is configured to individually switch the emission and the emission stop of the identification light 90 from each of the plurality of light output ports 12, but the present invention is not limited thereto. In the present invention, the light source 10 may simply emit the identification light 90 from all of the light output ports 12 at the same time. Even in the case where the identification light 90 is emitted from the plurality of light output ports 12 simultaneously, by differentiating the patterns of lighting-on and lighting-off of the identification light 90 as described above, the plurality of light guide members 20 connected to the plurality of light output ports 12 can be distinguished from each other. Further, the light source 10 may be configured to emit the identification light 90 from only one light output port 12 that received the selection and not emit the identification light 90 from the plurality of light output ports 12 simultaneously.

In the above-described embodiment, an example is shown in which in the case of simultaneously emitting the identification light 90 from the plurality of light output ports 12, the identification light 90 different in pattern of lighting-on and lighting-off in unit time is shown, but the present invention is not limited thereto. The wavelength (i.e., the color of the identification light 90) of the identification light 90 emitted from the plurality of light output ports 12 may be differentiated. For example, by emitting the identification light 90 in such a manner that PORT 1 is green, PORT 2 is yellow, PORT 3 is blue, and so on, the difference between the identification light 90 can be recognized.

In the above-described embodiment, an example is shown in which a plurality of first identification operation units 73a corresponding to a plurality of light output ports 12 is provided, but the present invention is not limited thereto. In the present invention, it may be configured not to provide a plurality of first identification operation units 73a.

In the above-described embodiment, an example is shown in which one second identification operation unit 73b is provided for a plurality of light output ports 12, but the present invention is not limited thereto. In the present invention, it may be configured not to provide the second identification operation unit 73b.

In the above-described embodiment, an example is shown in which the plurality of first identification operation units 73a and the second identification operation unit 73b are each configured by a button (GUI) displayed on the display unit 51, but the present invention is not limited thereto. In the present invention, the plurality of first identification operation units 73a may be composed of mechanical switches. Similarly, the second identification operation unit 73b may be composed of a mechanical switch.

Further, in the above-described embodiment, an example is shown in which it is further provided with the light detection unit 40 that accepts the emission end 22 of any one of the plurality of light guide members 20 to detect the light output from the emission end 22, but the present invention is not limited thereto. The light irradiation device 100 may not be required to have a light detection unit 40. In this case, it is sufficient to use a light detection device provided separately from the light irradiation device 100 to perform calibration processing.

In the above-described embodiment, an example is shown in which the controller 30 is configured to control the light source 10 to emit the identification light 90 when the calibration processing has been successfully completed, but the present invention is not limited thereto. In the present invention, it is not necessary to perform the control to emit the identification light 90 when the calibration processing has been successfully completed. The completion of calibration processing may be notified by a notification means other than the identification light 90. The notification means is, for example, an audio output.

In the above-described embodiment, an example is shown in which the display unit 51 and the first operation unit 52 are each composed of a touch panel display, but the present invention is not limited thereto. In the present invention, the display unit 51 and the first operation unit 52 may be configured separately by a display device and an input device, such as, e.g., a keyboard and a mouse. For example, it may be configured such that a display unit 51 composed of a liquid crystal display device or the like and a first operation unit 52, such as, e.g., a keyboard and a mouse, are provided in a single housing in which the controller 30 and light source 10 are built in.

Further, in the above-described embodiment, an example is shown in which the touch panel display configured by the display unit 51 and the first operation unit 52 is connected to the controller 30, but the present invention is not limited thereto. In the present invention, the display unit 51, the first operation unit 52, and the controller 30 may constitute a single tablet-type terminal or other information terminal.

### [Aspects]

It would be understood by those skilled in the art that the exemplary embodiments described above are specific examples of the following aspects.

### (Item 1)

A light irradiation device for irradiating a treatment target site of a subject to which a medicine containing a photosensitive substance has been administered with therapeutic light, the light irradiation device comprising:
a light source including a plurality of light output ports, the light source being configured to emit the therapeutic light and identification light from each of the plurality of light output ports, the identification light being lower in intensity than the therapeutic light;
a plurality of light guide members each having an incident end to be connected to one of the plurality of light output ports of the light source and an emission end to emit light incident from the incident end; and
a controller configured to control the light source,
wherein the controller is configured to perform control to cause the identification light to be emitted from each of the plurality of light output ports to light up the plurality of light guide members connected to the plurality of light output ports, respectively, in an identifiable manner.

### (Item 2)

The light irradiation device as recited in the above-described Item 1,
wherein the light source is configured to emit the therapeutic light of a first wavelength and the identification light of a second wavelength from each of the plurality of light output ports, the second wavelength being different from the first wavelength.

### (Item 3)

The light irradiation device as recited in the above-described Item 2,
wherein the second wavelength of the identification light is a wavelength outside a first bandwidth of light-shielding glasses that block light in the first bandwidth including the first wavelength of the therapeutic light, and the second wavelength is a wavelength within a range of visible light.

### (Item 4)

The light irradiation device as recited in the above-described Item 2,
wherein the light source includes a first light source for generating the therapeutic light and a second light source for generating the identification light and is configured to emit the therapeutic light and the identification light independently from the plurality of light output ports.

### (Item 5)

The light irradiation device as recited in the above-described Irem 2,
wherein the light source is configured to switch emission and emission stop of the identification light individually from each of the plurality of light output ports.

### (Item 6)

The light irradiation device as recited in the above-described Item 5, further comprising:
a plurality of first identification operation units provided corresponding to the plurality of light output ports to receive an operation input for instructing emission of the identification light for each light output port,
wherein the controller controls the light source to emit the identification light from the light output port corresponding to the first identification operation unit that received the operation input out of the plurality of first identification operation units.

### (Item 7)

The light irradiation device as recited in the above-described Item 2,
wherein the light source is configured to simultaneously emit the identification light mutually different in pattern of lighting-on and lighting-off in unit time from each of the plurality of light output ports.

### (Item 8)

The light irradiation device as recited in the above-described Item 7, further comprising:
a second identification operation unit, one piece of the second identification operation unit being provided for the plurality of light output ports, the second identification operation unit being configured to receive an operation input for instructing emission of the identification light from the plurality of light output ports,
wherein the controller controls the light source to cause the identification light mutually different in the pattern to be emitted from each of the plurality of light output ports according to the operation input to the second identification operation unit.

### (Item 9)

The light irradiation device as recited in the above-described Item 1,
wherein the light source includes a first light source for generating the therapeutic light of a first wavelength, and
wherein the identification light is light of the first wavelength generated from the first light source, the identification light being light lower in intensity than the therapeutic light.

### (Item 10)

The light irradiation device as recited in the above-described Item 1, further comprising:
a light detection unit configured to accept any one of emission ends of the plurality of light guide members to detect light output from the emission end,
wherein the controller is configured to perform calibration processing to adjust emission intensity of the therapeutic light at each of the plurality of light output ports based on a detection result of the light detection unit.

### (Item 11)

The light irradiation device as recited in the above-described Item 10,
wherein the controller is configured to
determine whether the calibration processing has been successfully completed based on a comparison between a detection result of the light detection unit and a set value of the emission intensity of the therapeutic light, and
perform control to cause the light source to emit the identification light when the calibration processing has been successfully completed.

### (Item 12)

A control method for a light irradiation device for irradiating a treatment target site of a subject to which a medicine containing a photosensitive substance has been administered with therapeutic light, the control method comprising:
a step of causing identification light lower in intensity than the therapeutic light to be emitted from each of the plurality of light output ports to which the plurality of light guide members is connected, respectively, to light up the plurality of light guide members connected to the plurality of light output ports in an identifiable manner; and
a step of causing the therapeutic light to be emitted from each of the plurality of light output ports.

### Description of Reference Symbols

- 10:: Light source
- 12:: Light output port
- 13a:: First light source
- 13b:: Second light source
- 20:: Light guide member
- 21:: Incident end
- 22:: Emission end
- 30:: Controller
- 40:: Light detection unit
- 60:: Light-shielding glasses
- 73a:: First identification operation unit
- 73b:: Second identification operation unit
- 80:: Therapeutic light
- 90:: Identification light
- 100:: Light irradiation device
- 101:: Patient (Subject)
- 101a:: Treatment target site
- 102:: Medicine
- 110:: Unit time

## Claims

1. A light irradiation device for irradiating a treatment target site of a subject to which a medicine containing a photosensitive substance has been administered with therapeutic light, the light irradiation device comprising:
a light source including a plurality of light output ports, the light source being configured to emit the therapeutic light and identification light from each of the plurality of light output ports, the identification light being lower in intensity than the therapeutic light;
a plurality of light guide members each having an incident end to be connected to one of the plurality of light output ports of the light source and an emission end to emit light incident from the incident end; and
a controller configured to control the light source,
wherein the controller is configured to perform control to cause the identification light to be emitted from each of the plurality of light output ports to light up the plurality of light guide members connected to the plurality of light output ports, respectively, in an identifiable manner.

2. The light irradiation device as recited in claim 1,
wherein the light source is configured to emit the therapeutic light of a first wavelength and the identification light of a second wavelength from each of the plurality of light output ports, the second wavelength being different from the first wavelength.

3. The light irradiation device as recited in claim 2,
wherein the second wavelength of the identification light is a wavelength outside a first bandwidth of light-shielding glasses that block light in the first bandwidth including the first wavelength of the therapeutic light, and the second wavelength is a wavelength within a range of visible light.

4. The light irradiation device as recited in claim 2,
wherein the light source includes a first light source for generating the therapeutic light and a second light source for generating the identification light and is configured to emit the therapeutic light and the identification light independently from the plurality of light output ports.

5. The light irradiation device as recited in claim 2,
wherein the light source is configured to switch emission and emission stop of the identification light individually from each of the plurality of light output ports.

6. The light irradiation device as recited in claim 5, further comprising:
a plurality of first identification operation units provided corresponding to the plurality of light output ports to receive an operation input for instructing emission of the identification light for each light output port,
wherein the controller controls the light source to emit the identification light from the light output port corresponding to the first identification operation unit that received the operation input out of the plurality of first identification operation units.

7. The light irradiation device as recited in claim 2,
wherein the light source is configured to simultaneously emit the identification light mutually different in pattern of lighting-on and lighting-off in unit time from each of the plurality of light output ports.

8. The light irradiation device as recited in claim 7, further comprising:
a second identification operation unit, one piece of the second identification operation unit being provided for the plurality of light output ports, the second identification operation unit being configured to receive an operation input for instructing emission of the identification light from the plurality of light output ports,
wherein the controller controls the light source to cause the identification light mutually different in the pattern to be emitted from each of the plurality of light output ports according to the operation input to the second identification operation unit.

9. The light irradiation device as recited in claim 1,
wherein the light source includes a first light source for generating the therapeutic light of a first wavelength, and
wherein the identification light is light of the first wavelength generated from the first light source, the identification light being light lower in intensity than the therapeutic light.

10. The light irradiation device as recited in claim 1, further comprising:
a light detection unit configured to accept any one of emission ends of the plurality of light guide members to detect light output from the emission end,
wherein the controller is configured to perform calibration processing to adjust emission intensity of the therapeutic light at each of the plurality of light output ports based on a detection result of the light detection unit.

11. The light irradiation device as recited in claim 10,
wherein the controller is configured to
determine whether the calibration processing has been successfully completed based on a comparison between a detection result of the light detection unit and a set value of the emission intensity of the therapeutic light, and
perform control to cause the light source to emit the identification light when the calibration processing has been successfully completed.

12. A control method for a light irradiation device for irradiating a treatment target site of a subject to which a medicine containing a photosensitive substance has been administered with therapeutic light, the control method comprising:
a step of causing identification light lower in intensity than the therapeutic light to be emitted from each of the plurality of light output ports to which the plurality of light guide members is connected, respectively, to light up the plurality of light guide members connected to the plurality of light output ports in an identifiable manner; and
a step of causing the therapeutic light to be emitted from each of the plurality of light output ports.
